(19) Europäisches Patentamt
European Patent Office
Office européen des brevets

(11) Veröffentlichungsnummer: **0 000 893**
**B1**

(12) # EUROPÄISCHE PATENTSCHRIFT

(21) Anmeldenummer: **78100606.9**

(22) Anmeldetag: **07.08.78**

(51) Int. Cl³: **C 07 D 251/18,**
C 07 D 251/50,
C 07 D 251/52,
C 07 D 251/70,
C 08 G 18/79

(54) **Verfahren zur Herstellung von Polyisocyanaten und ihre Verwendung**

(30) Priorität: **19.08.77 DE 2737402**

(43) Veröffentlichungstag der Anmeldung:
**07.03.79 Patentblatt 79/05**

(45) Bekanntmachung des Hinweises auf die Patenterteilung:
**17.09.80 Patentblatt 80/19**

(84) Benannte Vertragsstaaten:
**BE DE FR GB**

(56) Entgegenhaltungen:
**DE - A - 1 943 635**
**DE - A - 2 436 740**
**DE - A - 2 507 682**
**DE - A - 2 653 408**
**DE - B - 2 032 547**
**FR - A - 2 243 936**

(73) Patentinhaber: **BAYER AG**
**Zentralbereich Patente, Marken und Lizenzen**
**D - 5090 Leverkusen 1, Bayerwerk (DE)**

(72) Erfinder: **Grögler, Gerhard, Dr.**
**von-Diergardt-Strasse 46**
**D - 5090 Leverkusen (DE)**
**Meyborg, Holger, Dr.**
**Bergstrasse 92**
**D - 5068 Odenthal-Gloebusch (DE)**

**EP 0 000 893 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur Herstellung von Polyisocyanaten und ihre Verwendung

Die Erfindung betrifft ein neues Verfahren zur Herstellung neuartiger Polyisocyanate, die als einbaufähige Füllstoffe bei der Herstellung von Polyurethankunststoffen geeignet sind, sowie die Verwendung der Verfahrensprodukte als einbaufähige Füllstoffe bei der Herstellung von Polyurethankunststoffen.

Es wurde gefunden, daß Melamin und andere Aminogruppen enthaltende s-Triazinderivate mit bestimmten, nachstehend näher beschriebenen Diisocyanaten unter bestimmten, nachstehend näher beschriebenen Reaktionsbedingungen zu neuen Harnstoffgruppen aufweisenden Polyisocyanaten umgesetzt werden können, ohne daß eine Kettenverlängerungsreaktion zu entsprechenden Polyharnstoffen eintritt.

Bei Melamin und anderen Aminogruppen enthaltenden Triazinen handelt es sich im Unterschied zu den überlicherweise bei der Herstellung von Harnstoffgruppen aufweisenden Polyisocyanaten eingesetzten Polyaminen um Polyamide — Melamin kann formal als das Triamid der Cyanursäure angesehen werden — die bekanntlich gegenüber Isocyanaten eine ganz wesentlich reduzierte Reaktivität aufweisen. Irgendwelche Analogieschlüsse von den bekannten Verfahren zur Herstellung von Harnstoffgruppen aufweisenden Polyisocyanaten durch Umsetzung von Polyaminen mit Polyisocyanaten (vgl. z.B. DE—AS 2 032 547) auf die Herstellung von Harnstoffgruppen (und s-Triazingruppen) aufweisenden Polyisocyanaten, wie sie nachstehend erstmals beschrieben ist, sind daher schon aus diesem Grund nicht möglich. Außerdem weisen die erfindungsgemäßen Verfahrensprodukte eine Reihe von bemerksenswerten Eigenschaften auf, die den üblichen Polyisocyanaten der Polyurethanchemie inklusive den beispielsweise aus der genannten Vorveröffentlichung bekannten Lösungen von Harnstoffpolyisocyanaten in unmodifizierten Polyisocyanaten nicht innewohnen. So sind die nachstehend näher beschriebenen Verfahrensprodukte in organischen Medien weitgehend schwerbis unlöslich und stellen daher und wegen ihres hohen Schmelzpunktes besonders interessante Polyisocyanate zur Herstellung spezieller Polyurethankunststoffe dar. Die Verfahrensprodukte eignen sich insbesondere als aktive Füllstoffe zur Herstellung von Polyurethanelastomeren, die erst bei erhöhter Temperatur mit den im Polyurethan vorliegenden aktiven Wasserstoffatomen abreagieren, so daß bei der Verwendung der Verfahrensprodukte beispielsweise als Dispersion in der zum Polyurethankunststoff eingesetzten Isocyanatkomponente eine selektive Aushärtung durch eine selektive Temperaturführung ermöglicht wird. So kann beispielsweise unter Verwendung von Dispersionen der erfindungsgemäßen Verfahrensprodukte in organischen Diisocyanaten in an sich bekannter Weise ein thermoplastisches Polyurethan hergestellt werden, welches thermoplastisch verformbar ist und durch eine Temperung bei erhöhter Temperatur in einen Duroplasten überführt werden kann. Durch den Einbau der erfindungsgemäßen Verfahrensprodukte in die Polyurethankunststoffe wird im übrigen deren Flammwidrigkeit wesentlich verbessert.

Gegenstand der Erfindung ist somit ein Verfahren zur Herstellung von Harnstoff- und s-Triazingruppen aufweisenden Polyisocyanaten durch Umsetzung von Aminogruppen aufweisenden organischen Verbindungen mit Diisocyanaten in einem molaren $NCO/NH_2$-Verhältnis von mindestens 2:1 das dadurch gekennzeichnet ist, daß man

a) als Aminogruppen aufweisende organische Verbindungen einen Chlor-, Brom-, $C_1$—$C_8$-Alkyl-, $C_1$—$C_8$-Alkoxy-, $C_6$—$C_{10}$- Aryl- oder $C_6$—$C_{10}$-Aroxy-Substituenten aufweisende Diamino-s-triazine oder Melamin und

b) als Diisocyanate aromatische Diisocyanate des Molekulargewichtsbereichs 174 bis 400, die neben einer sterisch ungehinderten aromatisch gebundenen Isocyanatgruppe eine weitere aromatisch gebundene Isocyanatgruppe aufweisen, welche durch mindestens einen Substituenten in o-Stellung sterisch gehindert ist, gegebenenfalls im Gemisch mit bis zu 50 Gew.-%, bezogen auf das Gesamtgemisch, an aromatischen Diisocyanaten mit Isocyanatgruppen gleicher Reaktivität verwendet.

Gegenstand der Erfindung ist auch die Verwendung der beim erfindungsgemäßen Verfahren erhaltenen Harnstoff- und s-Triazingruppen aufweisenden Polyisocyanate als einbaufähige Füllstoffe bei der Herstellung von Polyurethankunststoffen.

Für das erfindungsgemäße Verfahren geeignete aromatische Diisocyanate sind solche des Molekulargewichtsbereichs 174—400, welche neben einer freien, d.h. sterisch ungehinderten aromatisch gebundenen Isocyanatgruppe eine weitere aromatisch gebundene Isocyanatgruppe aufweisen, welche durch mindestens einen Substituenten in o-Stellung sterisch gehindert ist. Derartige Substituenten, die zu einer sterischen Hinderung der Isocyanatgruppe führen können, sind insbesondere $C_1$—$C_8$-Alkyl-, $C_6$—$C_{10}$-Aryl-, $C_1$—$C_8$-Alkoxy-, $C_1$—$C_8$-Thioäther-, $C_1$—$C_8$-Alkoxycarbonyl-, Chlor-, Brom-, oder Cyano-Gruppen. Des weiteren ist eine sterische Hinderung der aromatisch gebundenen Isocyanatgruppen dann gegeben, wenn das Grundgerüst des Diisocyanats ein ggf. über Brückenglieder wie Alkylen-, Äther-, Thioäther-, Sulfoxid- oder Sulfongruppen verknüpftes System mehrerer aromatischer Ringe darstellt und die (sterisch

gehinderte) Isocyanatgruppe in ortho-Stellung zu dem 2 aromatische Ringe verknüpfenden Brückenglied angeordnet ist.

Geeignete aromatische Diisocyanate sind demzufolge beispielsweise solche der allgemeinen Formeln I und II.

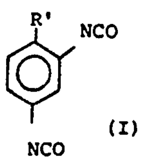
(I)

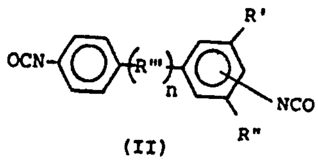
(II)

wobei

R′ und R″ für gleiche oder verschiedene Reste stehen und eine die sterische Hinderung der Isocyanatgruppe bewirkende Gruppe der oben beispielhaft genannten Art darstellen, wobei bei den Produkten der allgemeinen Formel II einer der Reste R′ bzw. R″ auch für Wasserstoff stehen kann, bzw. wobei in der allgemeinen Formel II auch beide R′ und R″ für Wasserstoff stehen können, falls die Isocyanatgruppe orthoständig zur Brücke R‴ bzw. im Falle von n = 0 ortho-ständig zum linken aromatischen Ring angeordnet ist;

R‴ für eine die aromatischen Ringe verknüpfende Brücke der oben beispielhaft genannten Art und

n für 0 oder 1 steht.

Es ist auch möglich, beim erfindungsgemäßen Verfahren Gemische aromatischer Diisocyanate einzusetzen, in welchen aromatische Diisocyanate mit Isocyanatgruppen gleicher Reaktivität wie beispielsweise 4,4′-Diisocyanatodiphenylmethan oder 2,6-Diisocyanatotoluol vorliegen, falls der Anteil derartiger Diisocyanate mit Isocyanatgruppen gleicher Reaktivität eine obere Grenze von 50, vorzugsweise von 40 Gew.-%, bezogen auf das Gesamtgemisch, nicht übersteigt.

Zu den besonders bevorzugten, für das erfindungsgemäße Verfahren geeigneten Diisocyanaten gehören z.B. 2,4-Diisocyanatotoluol, gegebenenfalls im Gemisch mit bis zu 50 Gew.-% bezogen auf Gesamtgemisch an 2,6 - Diisocyanatotoluol, 2,4′ - Diisocyanatodiphenylmethan, gegebenenfalls im Gemisch mit bis zu 50 Gew.-% bezogen auf Gesamtgemisch an 4,4′-Diisocyanatodiphenylmethan. Weiterhin geeignet sind z.B. 2,4′ - Diisocyanatodiphenylpropan, 2,4′ - Diisocyanatodiphenylsulfid, 2,4′ - Diisocyanatodiphenyläther, 2,4′ - Diisocyanatodiphenylsulfon, 2,4′ - Diisocyanatodiphenylsulfodioxid, 3 - Methyl - 4,4′ - diisocyanatodiphenylmethan, 3 - Äthyl - 4,4′ - diisocyanatodiphenylmethan, 3 - Isopropyl - 4,4′ - diisocyanatodiphenylmethan, 3,5 - Dimethyl - 4,4′ - diisocyanatodiphenylmethan, 3,5 - Diäthyl - 4,4′ - diisocyanatodiphenylmethan oder 3,5 - Diisopropyl - 4,4′ - diisocyanatodiphenylmethan, 3 - Carboxymethyl - 4,4′ - diisocyanatodiphenylmethan und 3 - Carboxyäthyl - 4,4′ - diisocyanatodiphenylmethan.

Für das erfindungsgemäße Verfahren geeignete Aminogruppen aufweisende organische Verbindungen sind neben Melamin alle beliebigen, obiger Definition entsprechende Diamino-s-triazine wie z.B. 6-Chlor-2,4-diamino-s-triazin, Benzoguanamin, Acetoguanamin oder Caprinoguanamin. Melamin ist besonders bevorzugt.

Bei der Durchführung des erfindingsgemäßen Verfahrens kommen die Reaktionspartner in solchen Mengenverhältnissen zum Einsatz, daß für jede Aminogruppe des s-Triazinderivats mindestens 2 Isocyanatgruppen des Diisocyanats im Reaktionsgemisch vorliegen, wobei sich diese Mengenangabe im Falle der Verwendung der obengenannten Gemische ausschließlich auf das Diisocyanat mit NCO-Gruppen unterschiedlicher Reaktivität bezieht. Im allgemeinen wird das s-Triazinderivat in einem Überschuß des vorgelegten diisocyanats eingerührt und die hierbei entstehende Lösung bzw. Dispersion anschließend auf 60—180°C, vorzugsweise 100—160°C, gegebenenfalls unter Zusatz bekannter Urethanisierungskatalysatoren, wie Zinkacetylacetonat, erhitzt, bis der Isocyanatgehalt des Reaktionsansatzes den berechneten Abfall zeigt. Das erfindungsgemäße Verfahrensprodukt fällt hierbei als im überschüssigen Diisocyanat unlöslicher Niederschlag aus.

Nach Abkühlung des Reaktionsansatzes auf Raumtemperatur können die erfindungsgemäßen Verfahrensprodukte durch Filtration und Waschen, beispielsweise mit einem inerten Lösungsmittel wie Aceton, Chloroform oder Petroläther, in reiner Form erhalten. Die geschilderte Arbeitsweise stellt die mit Abstand bevorzugte Verfahrensweise dar, da bei Verwendung eines $NCO/NH_2$-Äquivalentverhältnisses von $\geqslant$ 2:1, beispielsweise in Gegenwart inerter Lösungsmittel im allgemeinen keine erfindungsgemäßen Verfahrensprodukte, sondern hochvernetzte Umsetzungsprodukte erhalten werden. (Die Mitverwendung von die Verfahrensprodukte nicht lösenden unpolaren Verdünnungsmittel wie z.B. Lösungsbenzinen oder aromatischen Kohlenwasserstoffen wäre allerdings möglich, ist im allgemeinen jedoch überflüssig). Im allgemeinen wird der überschuß der Diisocyanat-Komponente hierbei so gewählt, daß im Reaktionsgemisch für jedes $H_2N$-Äquivalent des s-Triazin-Polyamins 3—10 vorzugsweise 5—8 NCO-Äquivalente bezogen auf alle Diisocyanate der Diisocyanat-Komponente vorliegen.

Die neuen erfindungsgemäßen Verfahrensprodukte sind in praktisch allen gängigen organischen Lösungsmitteln schwer- bis unlöslich, ausgenommen z.B. hochpolare, wie Dimethylformamid oder Dimethylsulfoxid. Die neuen Verbindungen sind hochschmelzend und zersetzen sich in der Schmelze. Sie eignen sich als Isocyanatkomponente in Pulverlacken auf Polyurethanbasis und insbesondere, wie bereits ausgeführt, als aktive Füllstoffe bei der Herstellung von Polyurethankunststoffen nach dem an sich bekannten Isocyanat-Polyadditionsverfahren. Bei dieser bevorzugten Verwendung der erfindungsgemäßen Verfahrensprodukte werden diese vorzugsweise in der zur Herstellung des Polyurethankunststoffes eingesetzten Isocyanat-Komponente dispergiert. Derartige Dispersionen der erfindungsgemäßen Verfahrensprodukte in organischen Polyisocyanaten werden dann in an sich bekannter Weise mit Polyhydroxypolyäthern und/oder Polyhydroxypolyestern gegebenenfalls in Kombination mit an sich bekannten Kettenverlängerungsmitteln zu Polyurethankunststoffen, insbesondere Polyurethanelastomeren, verarbeitet, die sich dann dank der Mitverwendung der erfindungsgemäßen Verbindungen durch eine verbesserte Wärmestandfestigkeit, eine verbesserte Weiterreißfestigkeit, eine verbesserte Bruchdehnung und eine verbesserte Zugfestigkeit auszeichnen.

Falls die Verwendung der erfindungsgemäßen Verbindungen zur Herstellung derartiger verbesserter Polyurethankunststoffe beabsichtigt ist, erübrigt sich oft ein Entfernen des bei der Herstellung der erfindungsgemäßen Verfahrensprodukte im Überschuß eingesetzten Diisocyanats. Vielmehr kann die bei der erfindungsgemäßen Umsetzung anfallende Dispersion der erfindungsgemäßen Verfahrensprodukte im im Überschuß eingesetzten Ausgangsdiisocyanat direkt zur Herstellung der Polyurethankunststoffe eingesetzt werden. Gemäß einer bevorzugten Ausführungsform der erfindungsgemäßen Verwendung der erfindungsgemäßen Verbindungen werden die zuletzt genannten Dispersionen mit den in der Polyurethanchemie an sich bekannten Polyätherpolyolen oder Polyesterpolyolen zu NCO-Gruppen aufweisenden Präpolymeren umgesetzt und zwar in solchen Mengenverhältnissen, daß in den entstehenden NCO-Präpolymeren sowohl vom unmodifizierten Diisocyanat herrührende Isocyanatgruppen als auch die Isocyanatgruppen der erfindungsgemäßen Verbindungen vorliegen. Diese NCO-Präpolymeren werden dann mit Kettenverlängerungsmitteln beispielsweise einem Diamin der in Beispiel 10 beispielhaft genannten Art zum hochmolekularen Polyurethan-Polyharnstoff umgesetzt, wobei die Menge des Kettenverlängerungsmittels entweder so gewählt wird, daß für alle Isocyanatgruppen des Präpolymeren inkl. den Isocyanatgruppen der dispergierten erfindungsgemäßen Verbindungen in erster Näherung eine Isocyanatreaktive Gruppe des Kettenverlängerungsmittels zur Verfügung steht oder aber so, daß in erster Näherung lediglich für die Isocyanatgruppen des Präpolymeren, welche nicht von den erfindungsgemäßen Verbindungen herrühren, Isocyanat-reaktive Gruppen des Kettenverlängerungsmittels zur Verfügung stehen, wobei ein nachträglicher chemischer Einbau der zunächst dispergierten erfindungsgemäßen Verbindungen durch Tempern der Umsetzungsprodukte bei 100 — 150°C erreicht wird (vgl. Beispiel 10). Insbesondere im erstgenannten Falle der Verwendung in etwa äquivalenter Mengen des Kettenverlängerungsmittels bezogen auf die Gesamtmenge der vorliegenden Isocyanatgruppen empfiehlt sich die Mitverwendung der bekannten Polyurethan-Katalysatoren wie z.B. Dibutylzinndilaurat, Zinndioctoat, Diazobicyclooctan und/oder Dimethyltetrahydropyrimidin.

Beispiel 1

Eine Mischung aus 1000 g (5,75 Mol) Toluylendiisocyanat (2,4- und 2,6-Isomere im Verhältnis 80:20) und 72,5 g (0,575 Mol) Melamin werden unter kräftigem Rühren 3 Stunden auf 120 bis 130°C erhitzt. Nach dem Abkühlen der Reaktionsmischung wird das Reaktionsprodukt abgesaugt und mit Aceton isocyanatfrei gewaschen. Nach Trocknung im Vakuum fallen 373 g eines farblosen, in organischen Lösungsmitteln extrem schwerlöslichen Produkts an. Ausbeute, berechnet, auf ein Additionsprodukt von Melamin und Toluylendiisocyanat im Mol-Verhältnis 1:3, 98%, Fp=350—380°C (Z).

Analyse: MG 648 $C_{30}H_{24}N_{12}O_6$

| | C | H | N |
|---|---|---|---|
| Berechnet: | 55,4 | 3,7 | 26,0 |
| Gefunden: | 55,6 | 3,7 | 26,3. |

Das Triisocyanat reagiert in der Kälte (RT) nur langsam mit aliphatischen Aminen wie Di-n-butylamin. Der NCO-Wert ist deshalb bei RT erst nach längerer Reaktionszeit feststellbar:

| Titrationszeit (min) | NCO (%) |
|---|---|
| 15 | 5,5 |
| 30 | 10,8 |
| 60 | 14,7 |
| 120 | 19,0 |

Wenn die Titration bei 50—70°C durchgeführt wird, findet man hingegen schon nach 3 bis 5 Minuten einen NCO-Wert von 19,3% (berechnet 19,5%).

Beispiel 2 (Vergleich)

Eine Mischung aus 63 g (0,5 Mol) Melamin und 313 g (1,8 Mol) des Toluylendiisocyanat des Beispiels 1 in 1 l Nitrobenzol wird auf 140°C erhitzt. Nach 45 Minuten ist der

Reaktionsansatz nicht mehr rührbar. Er wird mit Aceton verdünnt und abgesaugt. Der getrocknete Niederschlag weist einen NCO-Wert von 2,5% auf (berechnet 19,5%).

Beispiel 3 (Vergleich)

31,5 g (0,25 Mol) Melamin und 480 g (3 Mol) 1,3-Phenylendiisocyanat werden 7 Stunden unter kräftigem Rühren auf 100—140°C erhitzt. Anschließend wird der Reaktionsansatz mit Chloroform verdünnt und abgesaugt. Der im Vakuum getrocknete Niederschlag weist einen NCO-Wert von 10,9% auf (berechnet 20,8%).

Beispiel 4

31,5 g (0,25 Mol) Melamin und 500 g (2 Mol) eines Diphenylmethandiisocyanats mit ca. 40% 4,4'-Isomeren und 60% 2,4'-Isomeren werden 4 Stunden unter kräftigem Rühren auf 100°C erhitzt. Anschließend wird der Reaktionsansatz mit Chloroform/Petroläther verdünnt und abgesaugt. Der Niederschlag wird im Vakuum getrocknet. Ausbeute 200 g (91%). Festpunkt 252—60°C.

Analyse (MG 876) $C_{48}H_{36}N_{12}O_6$

| | C | H | N |
|---|---|---|---|
| Berechnet: | 65,8 | 4,12 | 19,2 |
| Gefunden: | 65,4 | 3,90 | 18,0 |

NCO-Wert 13,9% (theoretisch 14,4%)

Beispiel 5 (Vergleich)

Eine Mischung aus 12,6 g (0,1 Mol) Melamin und 250 g (1 Mol) geschmolzenem 4,4'-Diphenylmethandiisocyanat wird auf 120°C erhitzt. Nach 30 Minuten ist der Reaktionsansatz fest.

Beispiel 6

7,6 g (0,06 Mol) Melamin und 252 g (1 Mol) 2,4'-Diisocyanatodiphenyläther werden 4 Stunden auf 100°C erhitzt. Anschließend wird der Reaktionsansatz mit Methylenchlorid/Petroläther verdünnt und abgesaugt. Der Niederschlag wird im Vakuum getrocknet. Ausbeute 48 g (91%). Festpunkt 265—68°C.

Analyse: (MG 882) $C_{45}H_{30}N_{12}O_9$

| | C | H | N |
|---|---|---|---|
| Berechnet: | 61,2 | 3,4 | 19,0 |
| Gefunden: | 59,8 | 3,1 | 18,5 |

NCO-Wert 13,1% (theoretisch 14,3%)

Beispiel 7

Zu 7,1 kg (40,8 Mol) 2,4-Toluylendiisocyanat werden bei RT unter Rühren 0,567 kg (4,5 Mol) Melamin zugesetzt. Das Reaktionsgemisch wird auf 125—130°C erhitzt. Nach 2 Stunden wird auf 80—85°C abgekühlt. Anschließend wird das Melamintriisocyanat in Form einer feinverteilten Aufschlämmung enthaltende überschüssige 2,4-Toluylendiisocyanat mit 27,31 kg (13,7 Mol) eines difunktionellen Polypropylenglykols (OH—Zahl 56) präpolymerisiert. Nach 4 bis 5 Stunden bei 80—85°C weist das Präpolymer einen NCO-Wert von 3,2-3,4% auf. Wird die NCO-Bestimmung in der Hitze oder in Dimethylsulfoxid-Lösung durchgeführt, wird ein NCO-Wert von 4,4—4,6% bestimmt, da dann auch der NCO-Gehalt des Melamin-Diisocyanat-Adduktes miterfaßt wird.

Der Feststoffanteil des Präpolymers beträgt 8,4%, seine Viskosität liegt bei 4500 cP (25°C).

Beispiel 8

14,4 g (0,11 Mol) Melamin und 336 g (1,34 Mol) eines Diphenylmethandiisocyanats mit ca. 40% 4,4'-Isomeren- und 60% 2,4'-Isomerenanteil werden 4 Stunden unter kräftigem Rühren auf 100°C erhitzt. Anschließend werden 1000 g eines difunktionellen Polyätherdiols (OH—Zahl 28) bei 80°C zugesetzt. Nach 3 Stunden bei 90°C weist das Präpolymer einen NCO-Wert von 3,6% auf. Sein Feststoffgehalt beträgt 7,4%.

Beispiel 9

27,5g (0,24 Mol) Äthylenglykolmonobutyläther in 100 ml Chlorbenzol werden mit 50 g (0,08 Mol) der im Beispiel 1 isolierten Verbindung vermischt. Der Reaktionsansatz wird auf 130°C erhitzt. Er ist nach 90 Minuten nicht mehr rührbar. Bei Zusatz von 0,5 g Dibutylzinndilaurat unter sonst gleichen Bedingungen ist der Reaktionsansatz bereits nach 20 Minuten nicht mehr rührbar, bei Zusatz von 0,5 g Dimethyltetrahydropyrimidin schon nach 14 Minuten. Das Triisocyanat reagiert also unter den üblichen Bedingungen zum Urethan ab.

Beispiel 10

100 Gew.-Teile des in Beispiel 7 hergestellten, modifizierten NCO-Präpolymeren werden bei 60 bis 80°C im Vakuum entgast und anschließend mit 6 Gew.-Teilen 3,5-Diäthyl-1-methyl-2,4-diaminobenzol innerhalb von 30 Sekunden verrührt. Je die Hälfte des Reaktionsansatzes wird dann in eine auf 110 bzw. 130°C heiße Metallform gegossen. Die Gießzeit beträgt ca. 2 Minuten. Nach 4 bis 5 Minuten kann der Gießling entformt werden. Nach Temperung bei 110°C (24 Stunden) und bei 130°C werden jeweils die mechanischen Eigenschaften des Elastomers bestimmt:

| | Tempertemperatur | |
|---|---|---|
| | 110°C | 130°C |
| Zugfestigkeit (DIN 53504) | 7,5 MPa | 14,0 MPa |
| Bruchdehnung (DIN 53504) | 350% | 650% |
| Weiterreißwiderstand (DIN 53515) | 170 N | 230 N |
| Shore Härte A (DIN 53505) | 77 | 76 |
| Elastizität (DIN 53512) | 60 | 60 |

Aus der Zunahme der Zugfestigkeit, Bruchdehnung und Weiterreißfestigkeit bei gleicher Härte ist erkennbar, daß erst nach Temperung des Elastomeren bei ⩾ 130°C der Füllstoff in die PU-Matrix eingebaut wird, was in dem verstärkenden Effekt deutlich sichtbar wird.

**Patentansprüche**

1. Verfahren zur Herstellung von Harnstoff- und s-Triazingruppen aufweisenden Polyisocyanaten durch Umsetzung von Aminogruppen aufweisenden organischen Verbindungen mit Diisocyanaten in einem molaren $NCO/NH_2$-Verhältnis von mindestens 2:1, dadurch gekennzeichnet, daß man

a) als Aminogruppen aufweisende organische Verbindungen einen Chlor-, Brom-, $C_1$—$C_8$-Alkyl-, $C_1$—$C_8$-Alkoxy-, $C_6$—$C_{10}$-Aryl-oder $C_6$—$C_{10}$-Aroxy-Substituenten aufweisende Diamino-s-triazine oder Melamin und

b) als Diisocyanate aromatische Diisocyanate des Molekulargewichtsbereichs 174 bis 400, die neben einer sterisch ungehinderten aromatisch gebundenen Isocyanatgruppe eine weitere aromatisch gebundene Isocyanatgruppe aufweisen, welche durch mindestens einen Substituenten in o-Stellung sterisch gehindert ist, gegebenenfalls im Gemisch mit bis zu 50 Gew.-%. bezogen auf das Gesamtgemisch, an aromatischen Diisocyanaten mit Isocyanatgruppen gleicher Reaktivität verwendet.

2. Verfahren gemäß Anspruch 1, dadurch gekennzeichnet, daß man als Aminogruppen aufweisende Verbindung Melamin und als Diisocyanat 2,4-Diisocyanatotoluol, gegebenenfalls im Gemisch mit 2,6-Diisocyanatotoluol und/oder 2,4'-Diisocyanatodiphenylmethan, gegebenenfalls im Gemisch mit 4,4'-Diisocyanatodiphenylmethan verwendet.

3. Verwendung der gemäß Anspruch 1 und 2 erhaltenen Harnstoff- und s-Triazingruppen aufweisenden Polyisocyanate als einbaufähige Füllstoffe bei der Herstellung von Polyurethankunststoffen.

**Claims**

1. A process for the preparation of polyisocyanates containing urea and s-triazine groups by the reaction of organic compounds containing amino groups with diisocyanates in a molar $NCO/NH_2$ ratio of at least 2 : 1, characterized in that

a) melamine or diamine-s-triazines containing a chlorine, bromine, $C_1$—$C_8$-alkyl, $C_1$—$C_8$-alkoxy, $C_6$—$C_{10}$-aryl or $C_6$—$C_{10}$-aroxy substituent are used as the organic compounds containing amino groups and

b) aromatic diisocyanates having a molecular weight of from 174 to 400 which, in addition to a sterically unhindered, aromatically bound isocyanate group, contain another aromatically bound isocyanate group which is sterically hindered by at least one substituent in the *O*-position, optionally in admixture with up to 50% by weight, based on the mixture as a whole, of aromatic diisocyanates containing isocyanate groups of equal reactivity.

2. A process according to claim 1, characterized in that melamine is used as the compound containing amino groups and 2,4-diisocyanatotoluene, optionally in admixture with 2,6-diisocyanatotoluene and/or 2,4'-diisocyanatodiphenyl methane, optionally in admixture with 4,4'-diisocyanatodiphenyl methane, are used as the diisocyanate.

3. The use of the polyisocyanates containing urea- and s-triazine groups obtained according to claims 1 and 2, as incorporatable fillers in the production of polyurethane plastics.

**Revendications**

1. Procédé pour la préparation de polyisocyanates portant des groupes urée et s-triazine par réaction de composés organiques portant des groupes amino avec des diisocyanates dans un rapport molaire $NCO/NH_2$ d'au moins 2:1, caractérisé en ce que:

a) on utilise en tant que composé organique portant des groupes amino une diamino-s-triazine portant des substituants chloro-, bromo-, alkyle en $C_1$—$C_8$, alcoxy en $C_1$—$C_8$, aryle en $C_6$—$C_{10}$ ou aroxy en $C_6$—$C_{10}$, ou la mélamine, et

b) on utilise en tant que diisocyanates des diisocyanates aromatiques dont le poids moléculaire se situe dans l'intervalle de 174 à 400 et qui, en plus d'un groupe isocyanate à liaison aromatique ne faisant pas l'objet d'un empêchement stérique, portent un autre groupe isocyanate à liaison aromatique faisant l'objet d'un empêchement stérique par au moins un substituant en position ortho, éventuellement

en mélange avec une proportion allant jusqu'a à 50% en poids, par rapport au mélange total, de diisocyanates aromatiques dont les groupes isocyanate ont la même réactivité.

2. Procédé selon la revendication 1, caractérisé en ce que l'on utilise en tant que composé portant des groupes amino la mélamine et, en tant que diisocyanate, le 2,4-diisocyanato-toluène, éventuellement en mélange avec le 2,6-diisocyanato-toluène, et/ou le 2,4'-diisocyanato-diphénylméthane, éventuellement en mélange avec le 4,4'-diisocyanato-diphénylméthane.

3. Utilisation des polyisocyanates portant des groupes urée et s-triazine, obtenus selon les revendications 1 et 2, en tant que matières de charge incorporables chimiquement à la préparation de résines synthétiques de polyuréthannes.